# EUROPEAN PATENT APPLICATION

(11) **EP 1 271 144 A1**
(43) Date of publication of application: **02.01.2003**
(21) Application number: 01115427.5
(22) Date of filing: 27.06.2001
(51) Int. Cl.: G01N 33/487

(54) **Method and circuitry for controlling and measuring electrical parameters in a biological membrane**

(71) Applicant: flyion GmbH, 72076 Tübingen (DE)
(72) Inventor: Lepple-Wienhues, Albrecht, Dr., 72070 Tübingen (DE)
(74) Representative: Patentanwälte Ruff, Wilhelm, Beier, Dauster & Partner

(57) **Abstract**

The invention relates to a method for controlling and measuring electrical parameters in a biological membrane (M) with a measuring circuitry, wherein capacitances and series resistances of the measuring circuitry are digitally compensated and wherein the digital compensation is performed by a computer (PC) connected to the measuring circuitry via AD/DA converters (ADC, DAC, DAC1, DAC2), In one embodiment the measuring circuitry is an I-V-converter (IVC) connected to a biological membrane (M). An output of the I-V-converter is connected via an AD-converter (ADC) to the computer (PC) for analyzing the I-output signal. The computer (PC) calculates a V-input signal being input via a DA-converter (DAC, DAC1) to the measuring circuitry. By analysis of the output signals the electrical characteristics can be calculated and electronically compensated so that the entire measuring circuitry including the biological membrane shows the desired frequency/time response and an electrical signal from the membrane (M) and its embedded proteins in a biological experiment can be controlled and measured exactly.

## Description

The invention relates to a method as well as a circuitry for controlling and measuring electrical parameters through a biological membrane. Within the context of this specification the word "comprises" is taken to mean "includes" and is not intended to mean "is limited to only".

Electronic circuitry is used for measuring and controlling electrical parameters required in order to study and manipulate the function of membrane proteins (e.g. ion channels and carriers) that carry charged particles (e.g. ions) across the lipid membranes surrounding cells. Many pharmacological compounds act via modulation of ion channels, but the development of novel agents is hindered considerably by the difficulty of measuring at high troughput function and modulation of function.

Typical methods to study such proteins are the patch-clamp technique and the lipid bilayer technique. For a description and an exemplary method of the patch clamp technique see European Patent Application file No. 00116515.8 of July 31^{st}, 2000, which is being incorporated herein by explicit reference. The term "biological membrane" used herein includes cellular outer and inner membranes, membanes of subcellular organelles, artificial membranes such as lipid bilayers and other membranes known to a person skilled in the art. The term "membrane proteins" includes ion channels, other channels, transporters, receptors and proteins partially or completely embedded in the membrane or associated to the membrane.

For a typical measurement of electrical parameters in membrane proteins and membranes a device containing a metal electrode (e.g. a pipette) is connected to a lipid membrane surrounding a cell or another biological or artificial structure and the electrode is electrically coupled to an analog amplifier. That amplifier performs a sensitive current-to-voltage conversion at low noise and controls the voltage across the membrane in variable patterns. The amplifier typically contains a variety of analog electrical circuits consisting of operational amplifiers, capacitors and resistors.

Several artifacts typically disturb the measurement of electrical parameters in said membrane structures.

Because of the small geometric dimensions of biological membranes and the measuring devices connected to them, the capacitive charge movements can exceed the currents under observation. Therefore, special attention has been assigned to compensate for and cancel capacitive current transients resulting from changes in command voltage. Another important problem is the series access resistance between the membrane structure of interest and the recording electrode introducing voltage and current errors (e.g. in patch-clamp experiments in the whole-cell configuration).

The typical measuring amplifier employs three different circuitries to compensate for
- 1.: "fast" capacitance (resulting mainly from the measuring chamber, e.g. the patch pipette or from small membrane areas)
- 2.: "slow" capacitance (resulting mainly from larger biological membrane areas, e.g. "whole cell recordings") in series with a series resistance in the order of several MegaOhms
- 3.: series resistance (resulting from a narrow or limited access to the membrane)

These circuitries inject currents into the measuring probe through capacitors and change the command voltage time course in order to compensate for capacitive charge movements and distortions of the desired signal.

The analog circuitry allows either "voltage clamp" controlling a constant or variable voltage while measuring the current or "current clamp" controlling the current at a desired constant value or time pattern and measuring the voltage. Modern analog circuitry introduces very low noise levels mainly stemming from capacitive and thermal fluctuations, typically in the range of 100 fA (10-3000 Hz) RMS.

Major problems with the analog amplifier compensation circuitry are
- miniaturization for measurements in parallel channels is hindered by the large number of operational amplifiers and passive electronic elements required
- capacitive coupling, capacitive noise and crosstalk between the channels severely limits miniaturization and arrangement of multiple devices and circuits
- by design (with the use of single analog RC circuitry) only single RC combinations can be compensated
- only a limited range of RC values can be compensated

The present invention addresses the problems associated with the known measuring methods and measuring circuitry. The principal objectives of the present invention are;
- Miniaturization of the amplifier design by reducing the number of active and passive analog circuitry elements for parallel measurements in biological membranes using multiple electrodes
- Reduction of capacitive coupling, noise and crosstalk between electrodes by reducing the number of active and passive analog circuitry elements
- Compensation of complex combinations of capacitances and resistances frequently encountered when measuring electrical parameters in biological membranes
- Extending the range of capacitances / resistances that can be compensated

These problems are solved by a method with the features of claim 1 as well as by an electronic circuitry with the features of claim 18. Several embodiments of the invention are included in the subclaims and are described below. The invention describes a method and a digital electronic amplifier designed to measure and control in biological membranes voltages between -500 and +500 mV and currents ranging from a few hundred femtoamperes to hundreds of nanoamperes.

The advantages offered by the present invention are achieved by the replacement of conventional analog circuitry for correction of the frequency/transient response of the system. With the present invention this correction is performed instead by software on a computer, that in one embodiment reads current responses from the output of an operational amplifier via an AD converter and controls the voltage applied to the probe via a DA converter connected to another operational amplifier.

The software is designed to control the system in such a way that a preferred current response pattern, e.g. a square step, can be achieved by applying a complex voltage pattern that includes the required charging transients and time constants for a given stray capacitance/resistance combination. The software is also designed to estimate electrical parameters like stray capacitances, series resistances and combinations thereof by stepwise or continuous approximation and to optimize the preferred current response pattern.

With digital compensation of the capacitances and series resistances of the measuring circuitry performed by a computer connected to the measuring circuitry via AD/DA-converters it is possible to fulfill the following requirements:
- Miniaturization by reducing the number of active and passive analog circuitry elements
- Reduction of capacitive coupling, noise and crosstalk between channels
- Low electrical noise (typically 100 fA rms 10-3000 Hz)
- Simple gain switching (1-100 mV/pA)
- Simple compensation of a large range of capacitances and resistances
- Compensation of complex combinations of capacitances and resistances
- Rapid and exact forcing of the desired command voltage (voltage clamp)
- Measurement and compensation of capacitive charge movement in the measuring device and the membrane
- Compensation for current and voltage errors arising from series resistances and stray capacitances in the measuring device

These features are essential for assays measuring electrical properties of biological membrane proteins whose function typically depends on voltage and timing of voltage changes. The improvements achieved with the current invention are especially important for multichannel design of electrophysiological assays to perform multiple measurements in biological membranes at the same time.

In a preferred method the measuring circuitry is an I-V-converter connected to the biological membrane. An output of the I-V-Converter can be connected via an AD-converter to the computer for analyzing the I-output signal, wherein preferably the computer calculates a V-input signal being input via a DA-converter to the measuring circuitry.

In one embodiment the invention relates to a digital measuring system for measuring and controlling current and voltage across biological membranes. The invention uses digital compensation of capacitances and series resistances instead of analog circuitry. These improvements are preferably achieved in the invention by connecting an AD-converter (ADC) to the Imon output of the I-V converter and a DA-converter (DAC) to the Vcom input of the I-V converter. The ADC is connected to a computer, e.g. a personal computer. The same computer controls the DAC. The computer may generate a rectangular, trapezoidal or sinusoidal voltage pattern through the DAC. The computer samples the current response I-output signal of the biological element/electrode assembly through the ADC. The computer calculates the electrical parameters of the entire circuitry including the biological element. The computer then changes the U-input signal in a way to achieve the desired current response of the entire circuitry including the biological element. Preferably a U-input signal can be generated by the computer as a result of calculating the measuring circuitry characteristics.

The capacitive charge movements can typically be distinguished from currents carried by ionic fluxes by timing, voltage dependence and several other means known to a person skilled in the art.

In a preferred embodiment the I-output response of a biological membrane to a voltage change is assumed to follow Ohm's law with no ion transport proteins opened within a voltage range known to a person skilled in the art.

In a preferred embodiment both membranes of a cell are intact and are arranged serially. In this arrangement the capacitance introduced by the biological membrane is small, the resistance of the preparation is large and the digital compensation can compensate for capacitances and series resistances in the recording apparatus.

In a preferred embodiment the I-output response of a biological membrane to a voltage change is anticipated with ion transport proteins blocked. The ion transport proteins are preferably blocked by application of a solution blocking the transport protein. The solution preferably contains substances blocking the channel, e.g. heavy metal ions or pharmaceutical compounds known to a person skilled in the art. Following calculation/compensation of the measuring circuitry characteristics the blocking agents are removed by solution exchange.

In a preferred embodiment the I-output response of a biological membrane is anticipated with permeable ions removed from the solutions surrounding the membrane protein or removed from one side of the protein with the voltage forced to a value that brings the elctrochemical driving force close to 0.

In a preferred embodiment the I-output response of a biological membrane is anticipated with cofactors removed from the solutions surrounding the membrane protein or removed from one side of the protein so that opening of the ion transport protein is prevented, for example Ca2+ ions or other second messengers regulating the opening of the channel protein. Such cofactors required for the opening of ion transport proteins are known to a person skilled in the art.

In one embodiment a membrane containing a voltage gated ion channel is examined. The ion channel can be closed for calculation of the measuring circuitry characteristics by avoiding voltages in the V-input signal that open the channel, e.g. by maintaining the membrane hyperpolarized, or by keeping the V-input signal at levels that inactivate the channel, e.g. by maintaining a depolarizing voltage. The voltage patterns, solutions and reagents required for such maneuvers with voltage-dependent ion transport proteins or other ion transport proteins are known to a person skilled in the art.

In a preferred embodiment the V-input signal is optimized for easy detection or evaluation, for example a rectangular, trapezoidal or sinusoidal signal.

In a preferred embodiment the V-input signal and the I-output signal are compared in order to calculate changes over time in membrane capacitance, for example by changes in membrane area through membrane fusion or membrane retrieval. Preferably the V input signal is sinusoidal and the phase shift between input- and output signals is calculated by the software.

In a preferred embodiment the digitally calculated correction voltage signals may be input into the measuring circuitry for compensating capacitive currents caused by the membrane or the recording apparatus, respectively. The capacitances can be stray capacitances.

In a preferred embodiment a correction voltage signal can be generated by the computer and input via the DAC other connective elements like amplifiers or filters by means of a variable capacitance. The correction voltage signal will preferably be fed into the electrode contacting the solution surrounding the membrane.

With the invention the following measurements and compensations can be performed:
- Measurement and compensation of the capacitance of the recording device that holds the membrane, e.g. capacitance of a pipette or a flat perforated chip
- Measurement and compensation of capacitance of the biological membrane
- Measurement and compensation of capacitance of portions of the biological membrane
- Measurement and compensation of series resistance to the biological membrane
- Measurement and compensation of combinations of series resistances and capacitances for portions of the biological membrane.

Measurements can be replaced by approximations if a partial compensation is sufficient in order to measure the properties of the ion transport protein embedded in the membrane.

The invention allows analysis of electrical signals, analysis of the electrical characteristics of the measuring circuitry and the device holding the membrane, and analysis of electrical parameters of the membrane and embedded proteins. With the invention time and frequency responses of the measuring circuitry including the membrane can be varied and analyzed. With the invention complex arrangements of capacitive and resistive elements can be analyzed and compensated. With the invention electronic circuitry can be miniaturized and automated in order to perform multiple parallel electrical measurements in biological membranes. With the invention said analysis and compensation can be automated.

These and further features can be gathered from the claims, description and drawing and the individual features, both single and in combinations, can be implemented in an embodiment of the invention and in other fields and can represent advantageous, independently protectable constructions for which protection is claimed here. The subdivision of the application into individual sections and the subtitles in no way restrict the general validity of the statements made thereunder.

### Brief description of the drawings

Embodiments of the invention are described in greater detail hereafter relative to the following drawings:
- Fig. 1: shows a sketch of a probe containing a biological membrane, an I-V-converter, AD/DA converters and a computer; and
- Fig. 2: shows a variation of the I-V-converter in Fig. 1 with an additional amplifier.

### Detailed description of the embodiments

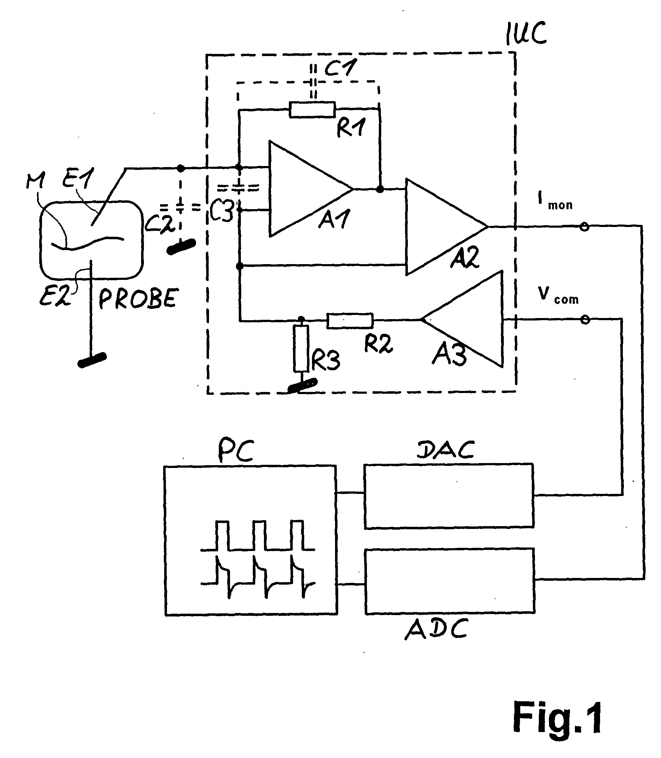

The digital measuring system illustrated in FIG. 1 comprises a measuring probe PROBE, preferably consisting of a biological membrane M in aqueous solution and two electrodes E1 and E2, preferably silver-silver/chloride electrodes. The biological membrane is placed in an electrolytic solution which is connected to system ground through E2 as reference electrode. The biological membrane is located in a chamber designed to provide high resistance parallel to that membrane, e.g. a patch-clamp pipette or a chamber for lipid bilayer techniques, see above European Patent Application Nr. 00 11 6515.8.

The electrode E1 is electrically connected to one input of an operational amplifier Al incorporated in a current-to-voltage converter circuitry IVC. The converter IVC includes a very high resistance feedback circuitry comprising a precision resistor R1 connecting the output of amplifier A1 back to the electrode E1. The other input to amplifier A1 is connected to a source of reference potential comprising an amplifier A3. The output of amplifier A3 is connected to the input of amplifier A1 through a series resistor R2, which is connected to system ground by a shunt resistor R3. The output of the reference voltage amplifier A3 is also connected to the shielding of the converter.

The output of the operational amplifier A1 in the current-to-voltage converter IVC is connected to an input of another differential amplifier A2. The second input to amplifier A2 is derived from the reference amplifier A3. The output of amplifier A2 represents the current monitor signal (Imon), representing the output signal, and is connected to an AD converter ADC. The digital output of the AD converter ADC is connected to a computer PC, in this case being a personal computer.

The input of the reference source amplifier A3 represents the command voltage (Vcom), representing the input signal. The input may comprise an operational amplifier and is connected to the analog output of a DA converter DAC, which is digitally controlled by the computer PC. A3 serves to apply a command voltage to the probe, for example a voltage step for the activation of an ionic channel in the biological membrane M.

In the circuitry illustrated in FIG. 1, the precision feedback resistor R1 may have a resistance of the order of ten gigaohms. Preferably, resistor R1 is a colloid film resistor with low capacitance selected for low noise, though other types may be used. Amplifier A1 may be a LF356 (National Semiconductor). Amplifier A1 may also consists of a preamplifier stage using a 0430 dual junction field-effect transistor (JFET), followed by a conventional integrated-circuit op-amp. The JFETs are characterized by low voltage noise and input capacitance and have small gate leakage currents. Amplifiers A2 and A3 may be Philips type NE 5534 or National Semiconductor type LF 356 selected for low voltage noise, especially above one kHz.

Distributed or "stray" capacitances are important in the operation of the system. One of these is the distributed feedback capacitance C1, which is of the order of 0.1 pF. The stray capacitance C2 between the electrode connection and ground depends on the probe containing the biological membrane and is in the range of 4-10 pF. C3 represents the input capacitance of amplifier A1 at approximately 4 pF as indicated above. A complex array of capacitances and resistances can occur in the PROBE depending on the area of the biological membrane, the geometry and material surrounding membrane and PROBE etc.

The improvement provided by the present invention comprises the replacement of conventional analog circuitry for correction of the frequency/transient response of the system. This correction is performed by a software on computer PC, that reads current responses from the output of amplifier Al via the AD converter ADC and controls the voltage applied to the probe via the DA converter DAC connected to amplifier A3. The software on computer PC is designed to control the system in such a way that a preferred current response pattern, e.g. a square step, can be achieved by applying a complex voltage pattern that includes the required charging currents and time constants for given stray capacitance/resistance combinations.

For comparison a typical state-of-the-art analog circuitry is depicted as described in "Design of the EPC-9, a computer controlled patch-clamp amplifier" by Sigworth, Journal of Neuroscience Methods 56 (1995), 195 - 202.

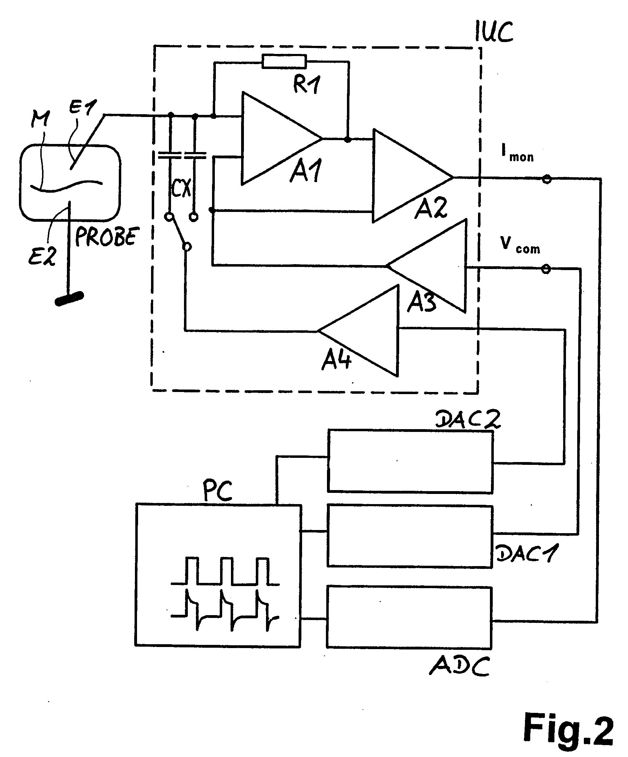

In Fig.2 another preferred embodiment of the digital measuring system is shown. The output of a second DAC2 is connected to the input of amplifier A4 via one or several different variable and exchangeable capacitors CX, e.g. 1 and 10 pF. To keep the input current within the measuring range, currents scaled by the software and put out via DAC2 can be injected through amplifier A4 to cancel capacitive currents for the charging of the pipette or chamber, biological membrane M, and stray capacitances.

### Description of a typical experiment:

A biological membrane, e.g. a cell membrane of a CHO cell expressing a transport protein, e.g. a voltage gated Na⁺ channel, is sealed to a chamber, e.g. a patch-clamp pipette in aqueous salt solution. The command voltage Vcom is continuously held "hyperpolarized" at -100 mV in order to avoid activation of the Na+ channel. Voltage steps to -105 and -95 mV of 10 ms duration are applied alternatively every 20 ms via DAC in Fig. 1. Due to stray capacitance and resistance combinations in the biological preparation the rectangular steps in the command voltage evoke overshooting, exponentially declining current spikes. These spikes are digitally read by the computer PC and the command voltage steps are distorted in order to "overcharge" the membrane M, that is a repetitive change of command voltage curves in order to approximate the current response to a rectangular response is performed by the software. Following approximation the computer PC can calculate the complex chain of resistors and capacitors and "cancelled" out the charging currents by distortion of the voltage command step.

Next a command voltage step from a holding potential of -90 mV to 40 mV is applied, opening the voltage-gated Na⁺ channel. The distortions of the command voltage are now scaled up to the magnitude of the voltage step. The current through the channel is now measured without charging currents and series resistance current/voltage errors.

In this example, opening of the Na⁺ channel is avoided during the measurement/ approximation of capacitive currents and cancellation by maintaining the membrane hyperpolarized. Alternatively, an ion transport protein can be blocked by choosing a useful aqueous solution either containing substances that block the channel or by omitting permeant molecules. Following the cancellation procedure the solution can be changed.

## Claims

1. Method for control and measurement of electrical parameters through a biological membrane (M) with a measuring circuitry, wherein capacitances and series resistances of the measuring circuitry (IVC) are digitally compensated, wherein the digital compensation is performed by a computer (PC) connected to the measuring circuitry via AD/DA-converters (ADC, DAC, DAC1, DAC2).

2. Method according to claim 1, wherein the measuring circuitry is an I-V-converter (IVC) connected to the biological membrane (M), an output of the I-V-converter being connected via an AD-converter (ADC) to the computer (PC) for analyzing the I-output signal, wherein preferably the computer calculates a V-input signal being input via a DA-converter (DAC, DAC1) to the measuring circuitry.

3. Method according to claim 2, wherein a V-input signal from one of the following group of signals is generated: rectangular, trapezoidal, sinusoidal.

4. Method according to one of the preceding claims, wherein the characteristics of the measuring circuitry (IVC), preferably of the biological Membrane (M) including its connections, are calculated by the computer (PC) by processing the I-output signal as a response to a typical V-input signal.

5. Method according to one of the preceding claims, wherein a V-input signal is generated by the computer (PC), preferably as a result of calculating the measuring circuitry (IUC) characteristics, the V-input signal. effecting a predetermined I-output signal from the measuring circuitry.

6. Method according to claim 5, wherein the predetermined I-output signal is optimized for being simply detected or evaluated, preferably being a rectangular signal.

7. Method according to one of the preceding claims, wherein digital correction voltage signals are input into the measuring circuitry (IUC) for compensating capacitive currents at the membrane (M) or the recording chamber or holder, respectively, the capacitances preferably being stray capacitances.

8. Method according to claim 7, wherein a correction voltage signal is generated by the computer (PC) and is input via a DA-converter (DAC, DAC1) and an amplifier (A4) by means of a variable or -switchable capacitance (CX), preferably being input to the electrode located near membrane (M).

9. Method according to one of the preceding claims, wherein the electric properties of a cell membrane or a ion transport protein in said membrane (M) are measured, wherein the membrane or the protein are inactivated during calculation of the measuring circuitry characteristics, the inactivation preferably being effected by maintaining the membrane hyperpolarized.

10. Method according to one of the preceding claims, wherein the electric properties of a cell membrane or a ion transport protein in said membrane (M) are measured, wherein the I-output response of a biological membrane to a voltage change is assumed to follow Ohm's law.

11. Method according to one of the preceding claims, wherein the electric properties of a cell membrane or a ion transport protein in said membrane (M) are measured, wherein both membranes of a cell are intact and are arranged serially, where the capacitance introduced by the biological membrane is small, the resistance of the preparation is large and the digital compensation can compensate for capacitances and series resistances in the recording apparatus.

12. Method according to one of the preceding claims, wherein the electric properties of a cell membrane or a ion transport protein in said membrane (M) are measured, wherein the I-output response of a biological membrane to a voltage change is anticipated with ion transport proteins blocked, where the ion transport proteins are preferably blocked by application of a solution blocking the transport protein, where the solution preferably contains substances blocking the channel, and where tollowing calculation/compensation of the measuring circuitry characteristics the blocking agents are removed by solution exchange.

13. Method according to one of the preceding claims, wherein the electric properties of a cell membrane or a ion transport protein in said membrane (M) are measured, wherein the I-output response of a biological membrane is anticipated with permeable ions removed from the solutions surrounding the membrane protein or removed from one side of the protein with the voltage forced to a value that brings the electrochemical driving force close to 0.

14. Method according to one of the preceding claims, wherein the electric properties of a cell membrane or a ion transport protein in said membrane (M) are measured, wherein the I-output response of a biological membrane is anticipated with cofactors removed from the solutions surrounding the membrane protein or removed from one side of the protein so that opening of the ion transport protein is prevented, for example Ca²⁺ ions or other second messengers regulating the opening of the channel protein.

15. Method according to one of the preceding claims, wherein the electric properties of a cell membrane or a ion transport protein in said membrane (M) are measured, wherein voltage-dependent membrane proteins are examined, wherein the membrane proteins are inactivated for calculation of the measuring circuitry characteristics by avoiding voltages in the V-input signal that activate the membrane proteins, e.g. by maintaining the membrane hyperpolarized, or by keeping the V-input signal at levels that inactivate the channel, e.g. by maintaining a depolarizing voltage.

16. Method according to one of the preceding claims, wherein the electric properties of a cell membrane or a ion transport protein in said membrane (M) are measured, wherein the V-input signal and the I-output signal are compared in order to calculate changes over time in membrane capacitance, for example by changes in membrane area through membrane fusion or membrane retrieval, wherein the V input signal is preferably sinusoidal and the phase shift between input- and output signals is calculated by the software.

17. Method according to one of the preceding claims, wherein the V-input signal and the I-output signal are compared in order to calculate changes over time in membrane capacitance, for example by changes in membrane area through membrane fusion or membrane retrieval. Preferably the V input signal is sinusoidal and the phase shift between input- and output signals is calculated by the software.

18. Measuring circuitry, preferably for control and measuring of electrical current through a biological membrane, comprising an I-V-converter circuitry (IVC), the I-V-converter having a connecting amplifier (A1) for connection to a probe, an input amplifier (A3) and an output amplifier (A2), wherein the input amplifier and the output amplifier are connected via DA/AD-converters (DAC, ADC) to a computer (PC).

19. Measuring circuitry according to claim 18, wherein the I-V-converter has a further amplifier (A4) with its output being connected via a switchable capacitor (CX) to the input of the connecting amplifier (A1), the input of this amplifier being connected via a second DA-converter (DAC2) to the computer (PC).
